# EUROPÄISCHE PATENTSCHRIFT

(11) **EP 2 709 577 B1**
(45) Veröffentlichungstag und Bekanntmachung des Hinweises auf die Patenterteilung: **21.06.2017**
(21) Anmeldenummer: 11726332.7
(22) Anmeldetag: 16.05.2011
(51) Int. Cl.: A61F 9/008, A61B 3/10, A61B 3/113

(54) **GERÄT ZUR UNTERSUCHUNG ODER BEARBEITUNG EINES HUMANEN AUGES**
DEVICE FOR EXAMINING OR TREATING A HUMAN EYE
APPAREIL POUR L'EXAMEN OU LE TRAITEMENT D'UN OEIL HUMAIN

(43) Veröffentlichungstag der Anmeldung: 26.03.2014
(73) Patentinhaber: WaveLight GmbH, 91058 Erlangen (DE)
(72) Erfinder: VOGLER, Klaus, 99444 Blankenhain (DE); ABRAHAM, Mario, 90559 Burgthann (DE)
(74) Vertreter: Katérle, Axel
(86) Internationale Anmeldenummer: PCT/EP2011/002414
(87) Internationale Veröffentlichungsnummer: WO 2012/155929

(56) Entgegenhaltungen:
- WO-A1-99/65431
- WO-A2-03/070090
- WO-A2-03/105678
- US-A- 5 644 642
- US-A1- 2006 187 462

## Beschreibung

Die Erfindung betrifft ein Gerät zur Untersuchung oder Bearbeitung eines humanen Auges.

Laserstrahlung kommt bei zahlreichen Techniken der Behandlung des menschlichen Auges zum Einsatz. Bei einigen dieser Techniken wird fokussierte Laserstrahlung dazu genutzt, Augengewebe zu ablatieren (abzutragen). Hierbei ist es nötig, den Strahlfokus kontrolliert auf das Auge zu richten, so dass die Ablation an der gewünschten Position des Auges stattfindet. Durch Bewegungen des Auges während der Behandlung kann sich aber das Auge gegenüber dem Behandlungs-Laserstrahl in seiner Position verändern. Dies kann dann zu einer Abweichung zwischen einer Soll-Position und einer Ist-Position der Ablation führen.

Aus diesem Grund ist es wünschenswert, die Bewegungen des Auges zu verfolgen und bei der Steuerung des Strahlfokus zu berücksichtigen. Zur Erfassung der Augenbewegungen bedient man sich eines Eye-Trackers. Derzeit ist zumeist ein zweidimensionales Eye-Tracking üblich, das auf der Erfassung des Pupillenrandes des Auges durch nur eine Kamera beruht. Aus dem Hell-Dunkel-Kontrastsprung am Pupillenrand (Iris) wird das Pupillenzentrum berechnet, das dann als Orientierungskoordinate für die Laserablation dient. Die Steuerung der Behandlungs-Laserstrahlung erfolgt dann unter Berücksichtigung der vom Eye-Tracker ermittelten Position des Pupillenzentrums. Jedoch liegt die Position des Pupillenzentrums nicht immer auf der Symmetrieachse des Auges oder der optischen Sehachse des Patienten (beispielsweise durch asymmetrische Verschiebung der Pupillenmitte bei unterschiedlicher Pupillengröße oder Abweichung von der Kreissymmetrie bei manchen Patienten). Eine solche Abweichung kann zu suboptimalen Behandlungsergebnissen führen.

Um solche aus der Positionsverschiebung der Pupillenmitte resultierende Ungenauigkeiten bei der Laserbehandlung zu vermeiden, kann das Pupillen-Tracking durch ein Limbus-Tracking ergänzt werden, das sich am unveränderlichen Hell-Dunkel-Übergang der weißen Sklera (Lederhaut des Auges) zur Iris orientiert. Markante Verschiebungen der Pupillenmitte können somit erkannt werden und als sogenannte Pupil-Center-Shift-Correction (PCSC) im Ablationsprogramm berücksichtigt werden.

Insgesamt nutzt der Stand der Technik bisher zur Verfolgung der Augenbewegung (Eye-Tracking) zwei-dimensionale Kamerabilderfassung. Daraus abgeleitete Positionsvorgaben können jedoch fehlerbehaftet sein, da die eigentlichen Augenbewegungen im drei-dimensionalen Raum erfolgen und somit durch drei Translationsbewegungen sowie drei Rotationsbewegungen beschrieben werden müssen. Desweiteren ermöglichen Eye-Tracker, die auf Kamera-basierten zwei-dimensionalen Bildaufnahmen beruhen, nur die indirekte Erfassung von drei-dimensionalen Daten durch rechenintensive Rekonstruktion. Es sind inzwischen auch kamerabasierte Eye-Tracker erhältlich, die ein fünf- oder sechs-dimensionales Eye-Tracking ermöglichen. Hierbei wird durch eine zusätzliche Projektion eines Lichtmusters aus Streifen auf das Auge und durch die Erfassung dieser Streifen (Registrierung der Krümmung, Lage und Verformung der Streifen) auf eine Orts- und Orientierungslage des Auges geschlossen. Das Registrierverfahren ist aber auch hier rechen- und zeitintensiv. Daher ist die Bildrate bisher verwendeter Eye-Tracker in ihrer Geschwindigkeit begrenzt und für eine Positionskorrektur bei der Behandlung des Auges mit Laserlicht oft zu langsam. Außerdem detektieren die hierfür benutzten Kamerasysteme lediglich vom Auge des Patienten gestreutes oder reflektiertes Licht, weswegen es notwendig ist, für entsprechende Beleuchtung des Auges zu sorgen (was sich jedoch auch auf die Behandlung störend auswirken kann) und gleichzeitig Lichteinfall von anderen Nebenbeleuchtungen aus dem Raum auf das Auge zu vermeiden.

Die WO 99/65431 betrifft ein Verfahren und eine Vorrichtung zum Tracken der Position einer Objektoberfläche. Die Vorrichtung ist als OCT Trackingvorrichtung ausgebildet, wobei ein Controller ein erfasstes Ausgangssignal verarbeitet und daraus ein Peak-Interferenz-Signal detektiert.

Die US 5,644,642 betrifft ein Verfahren und eine Vorrichtung zum Eye-Tracking unter Verwendung von optischer Kohärenz-Tomographie OCT. Dabei scannt eine OCT-Vorrichtung einen OCT-Strahl entsprechend einem beliebigen zwei-dimensionalen Scanpfad. Die Ausgabe der OCT-Vorrichtung wird als Eingabe in einem Computer zur Analyse bereitgestellt, um das Zentrum der Augenpupille oder eine grobe Pupillen-Irisgrenze zu bestimmen.

Die US 2006/0187462 betrifft ein Verfahren und eine Vorrichtung zur Verbesserung der Leistung und Genauigkeit der optischen Kohärenz-Tomographie OCT und beschreibt ein Scanning mit Bezug auf bestimmte Landmarken der Probe. Dabei werden OCT-Daten in einem ersten Scan und einem zweiten Scan erfasst, hieraus Landmarken des Objekts identifiziert und Veränderungen in deren Position ermittelt. Die Scans werden so durchgeführt, dass sie unterschiedliche transversale Querschnitte in der Probe messen, wobei als Scanmuster insbesondere unidirektionale Rasterscans, bidirektionale Scans oder axiale, an unterschiedlichen transversalen Positionen auf der Probe gemessene Scans zum Einsatz kommen.

Die WO 03/070090 betrifft ein Verfahren und eine Vorrichtung zur Durchführung einer OCT-Untersuchung eines Auges unter Verwendung eines Trackingsystems, um den OCT-Scanstrahl auf ein gewünschtes Merkmal in retinalem Gewebe zu arretieren.

Die WO 03/105678 betrifft ein Verfahren und eine Vorrichtung zur optischen Abbildung. Die optische Abbildung basiert auf optischer Kohärenz-Tomographie OCT, wobei zwei-dimensionale Bilder erzeugt werden und daraus ein drei-dimensionales Bild der Probe konstruiert wird. Eine ein-dimensionale OCT-Scantechnik kann benutzt werden, um zufällige Veränderungen des Abstands zwischen der Probe und der Abbildungsvorrichtung, d.h. axiale Bewegungen der Probe, zu tracken.

Eine Aufgabe der Erfindung ist es, ein Gerät zur Untersuchung oder Behandlung eines humanen Auges bereitzustellen, welches einen Eye-Tracker umfasst, der Ergebnisse über Augenbewegungen mit großer Schnelligkeit und Präzision liefern kann.

Diese Aufgabe wird mit einem Gerät nach Anspruch 1 und einem Verfahren nach Anspruch 4 gelöst.

Zur Lösung dieser Aufgabe ist erfindungsgemäß ein Gerät zur Untersuchung oder Behandlung eines humanen Auges vorgesehen, mit einem Eye-Tracker zur Erfassung von Augenbewegungen und zur Ausgabe eines für die erfassten Augenbewegungen repräsentativen Signals, wobei der Eye-Tracker eine zur zeitaufgelösten Erfassung von Schnittbildern des Auges eingerichtete interferometrische Bilderfassungseinrichtung, die auf Basis der drei-dimensionalen optischen Kohärenz-Tomographie arbeitet, sowie ein die Augenbewegungen allein aus den Schnittbildern ermittelndes Auswertemodul umfasst.

Die interferometrische Bilderfassungseinrichtung kann beispielsweise gekennzeichnet sein durch eine Scanrate von >200000 Linien-Scans pro Sekunde, weite laterale Scan-Bereiche >= 15mm, Tiefenbereiche >8 bis 12mm, digitale Hochgeschwindigkeitskameras (CCD, CMOS) mit bis zu 12000 Pixeln pro Linie oder mehr, hohe Auslesegeschwindigkeiten von etwa 140 kHz, hohe Nachweissensitivitäten von >-90dB und hohe Auflösungen im Bereich von <1 bis 10 µm. Drei-dimensionale Bildraten mit 500 frames pro Sekunde oder mehr sind möglich.

Die Erfindung hat den Vorteil, dass durch den Einsatz der interferometrischen Meßmethode sich der Lichteinfall von anderen Nebenbeleuchtungen aus dem Raum auf das Auge nicht störend auf das Eye-Tracking auswirkt, da nur kohärentes, nicht aber inkohärentes Licht in den Meßvorgang eingeht.

Die Erfindung ermöglicht es zudem, die hohe Auflösung, die hohe Messgeschwindigkeit sowie die hohe Sensitivität von interferometrischen Bilderfassungseinrichungen, die auf Basis der drei-dimensionalen optischen Kohärenz-Tomographie arbeiten, nicht nur wie bisher zur Tomographie, d.h. zur Strukturvermessung eines bestimmten Augenabschnitts, zu verwenden, sondern auch zur Messung der Lage und der Orientierung des Auges im Raum. Dabei ist entscheidend, dass für die Ermittlung der Position-, Orientierungs- und Bewegungssignale lediglich Daten von (einer oder mehreren) interferometrischen Bilderfassungseinrichtung(en) herangezogen werden, die jeweils eine Bilderfassung mit Zuordnung von X-, Y- und Z-Koordinate zu jedem Bildpunkt ermöglichen.

Mit Hilfe der zeitaufgelösten Erfassung der Schnittbilder des Auges ist es somit möglich, entsprechende Augenbewegungen zu ermitteln. Derartige Augenbewegungen schließen Zyklotorsionsbewegungen (ebene Drehungen um die optische Achse des Auges), Rollbewegungen (Drehungen des Auges um eine Achse senkrecht zur optischen Achse des Auges) sowie Translationsbewegungen in allen drei Raumrichtungen ein. Die Messgenauigkeit der sechs-dimensionalen Augenbewegungen kann aufgrund der Verwendung der interferometrischen Bilderfassungseinrichtungen deutlich höher sein als die eines auf Kameras basierten Eye-Trackers. Ebenso bietet das erfindungsgemäße Gerät eine deutlich höhere Geschwindigkeit bei der Erfassung der Augenbewegungen.

Gemäß einer Weiterbildung der Erfindung kann die interferometrische Bilderfassungseinrichtung dazu eingerichtet sein, mindestens zwei zueinander orthogonale Schnittbilder des Auges zu erfassen, die jeweils einen Schnitt im Wesentlichen ententlang der Sehachse des Auges repräsentieren. Die Schnittbilder können jedoch auch entlang der Sehachse des Auges, parallel zur Sehachse des Auges oder im Wesentlichen entlang der zentralen (optischen) Achse des Auges, entlang der zentralen Achse des Auges, parallel zur zentralen Achse des Auges oder am Apex des Auges orientiert verlaufen. Die zwei zueinander orthogonal angeordneten Schnittbilder erlauben eine Erfassung von Orientierungen und Positionen sowie von Roll- und Translationsbewegungen in X- und Y-Richtung (hierbei ist die übliche Notation zu verstehen, in der die Ausbreitungsrichtung des Messstrahls der interferometrischen Bilderfassungseinrichtung entlang Z verläuft und X und Y gemeinsam mit Z das drei-dimensionale karthesische Koordinatensystem komplettieren). Auch eine Translationsverschiebung und/oder -bewegung in Z-Richtung kann aus derartigen Schnittbildern bestimmt werden. Die Erfassung kann beispielsweise durch einen Vergleich mit einem oder mehreren zuvor (etwa vor Behandlungsbeginn) bestimmten Schnittbildern erfolgen. Insbesondere können hierfür hochaufgelöste drei-dimensionale Komplettschnittbilder zum Vergleich herangezogen werden.

Ferner ist die interferometrische Bilderfassungseinrichtung dazu eingerichtet, mindestens ein Schnittbild des Auges zu erfassen, das einen Schnitt entlang des Irisrandes des Auges repräsentiert. Anhand dieses Schnittbilds können dann markante Strukturen (beispielsweise innerhalb der Iris) identifiziert werden und für eine Bestimmung der Zyklotorsion des Auges genutzt werden. Ein derartiger Schnitt kann somit als enface Aufnahme des Auges verstanden werden.

Die interferometrische Bilderfassungseinrichtung und das Auswertemodul sind vorzugsweise dazu eingerichtet, eine Mehrzahl Schnittbilder des Auges zeitaufgelöst zu erfassen und aus den Schnittbildern eine zeitaufgelöste Topographie eines Teilbereiches des Auges als das für die Augenbewegung repräsentative Signal zu ermitteln. Die Mehrzahl Schnittbilder des Auges kann z.B. parallel zueinander versetzten Querschnitten des Auges entsprechen. Der Teilbereich des Auges kann z.B. die Kornea, die menschliche Linse, den Vorderkammerbereich, die Sklera, die Iris, den Apex der Kornea, die Linsenmitte der menschlichen Linse und/oder die Fovea ganz oder teilweise umfassen.

Die Erfindung ermöglicht es somit, anhand der zeitaufgelösten Topographie des Teilbereiches des Auges (z.B. anhand der zeitaufgelösten Topographie der Kornea) translatorische und rotatorische Bewegungen des Auges zu erfassen und gleichzeitig eine Behandlung des Auges an einem beliebigen Punkt innerhalb des Teilbereiches (z.B. am Apex der Kornea) zu orientieren. Der beliebige Punkt kann jedes ausgezeichnete Merkmal des Teilbereichs des Auges sein. Dies ermöglicht es, ein ausgezeichnetes Merkmal des Teilbereiches des Auges zu wählen, anhand dessen eine besonders genaue Orientierung und präzise Durchführung einer konkreten Behandlung des Auges möglich ist. So macht es z.B. Sinn, bei einer ablativen LaserBehandlung der Kornea den Apex der Kornea als ausgezeichnetes Merkmal zu wählen und daran zu orientieren. Eine Orientierung an der Pupille/Iris wie im Stand der Technik ist mit der Erfindung auch möglich, jedoch nicht zwingend erforderlich. Vorzugsweise umfasst das Gerät zur Untersuchung oder Bearbeitung eines humanen Auges ferner Komponenten zur Bereitstellung von fokussierter Behandlungs-Laserstrahlung und zum Richten derselben auf das Auge sowie eine Steueranordnung, die dazu eingerichtet ist, den Fokusort der Behandlungs-Laserstrahlung abhängig von dem für die erfassten Augenbewegung repräsentativen Signal einzustellen.

Die vorliegende Erfindung ermöglicht es somit, die aus den Schnittbildern gewonnenen Positionsdaten bestimmter Merkmale des Auges sowie die aus den Schnittbildern ermittelten Augenbewegungen bei der Behandlung des Auges zu berücksichtigen.

Merkmale des Auges können in diesem Zusammenhang beispielsweise der Apex der Kornea, ein Punkt auf der Innenseite der Kornea, der Pupillenmittelpunkt, der Mittelpunkt der menschlichen Linse oder die Fovea (Fleck des schärfsten Sehens) darstellen.

Die interferometrische Bilderfassungseinrichtung und die Steuereinrichtung können auch dazu eingerichtet sein, eine Abweichung eines Ist-Fokusortes der Behandlungs-Laserstrahlung von einem Soll-Fokusort der Behandlungs-Laserstrahlung am oder im Auge zu erkennen und ein Hinweissignal auszugeben, wobei die Steuereinrichtung bei Ausgabe des Hinweissignals z.B. die Emission der Behandlungs-Laserstrahlung auf das Auge unterbrechen oder stoppen kann.

Nach einem weiteren Gesichtspunkt ist erfindungsgemäß ferner ein Verfahren zur Untersuchung eines humanen Auges vorgesehen, umfassend die Schritte:
- Zeitaufgelöstes Erfassen von Schnittbildern des Auges auf Basis einer drei-dimensionalen optischen Kohärenz-Tomographie,
- Ermitteln von Augenbewegungen allein aus den Schnittbildern, und
- Ausgeben eines für die erfassten Augenbewegungen repräsentativen Signals.

Auch bei dem Verfahrensaspekt können beim Erfassen der Schnittbilder mindestens zwei zueinander orthogonale Schnittbilder des Auges erfasst werden, die jeweils einen Schnitt im Wesentlichen entlang der Sehachse des Auges repräsentieren. Ferner ist auch beim Verfahren denkbar, dass beim Erfassen der Schnittbilder mindestens ein Schnittbild des Auges erfasst wird, das einen Schnitt im Wesentlichen entlang des Irisrandes des Auges repräsentiert.

Auch ist vorstellbar, dass eine Mehrzahl Schnittbilder des Auges zeitaufgelöst erfasst wird und aus den Schnittbildern eine zeitaufgelöste Topographie eines Teilbereiches des Auges als das für die Augenbewegung repräsentative Signal ermittelt wird.

Die Erfindung ist durch die Ansprüche definiert und wird nachfolgend anhand der beigefügten Zeichnungen weiter erläutert, von denen
Figur 1 ein Ausführungsbeispiel eines Geräts zur Untersuchung oder Bearbeitung eines humanen Auges zeigt,
Figur 2 eine schematische Darstellung des menschlichen Auges im Schnitt zeigt,
Figur 3 eine schematische Darstellung eines Zentrierungsfehlers in Folge einer Augenrollbewegung zeigt, sowie
Figuren 4a und 4b schematische Darstellungen zum Verlauf von Schnittbildern im Auge zeigen.

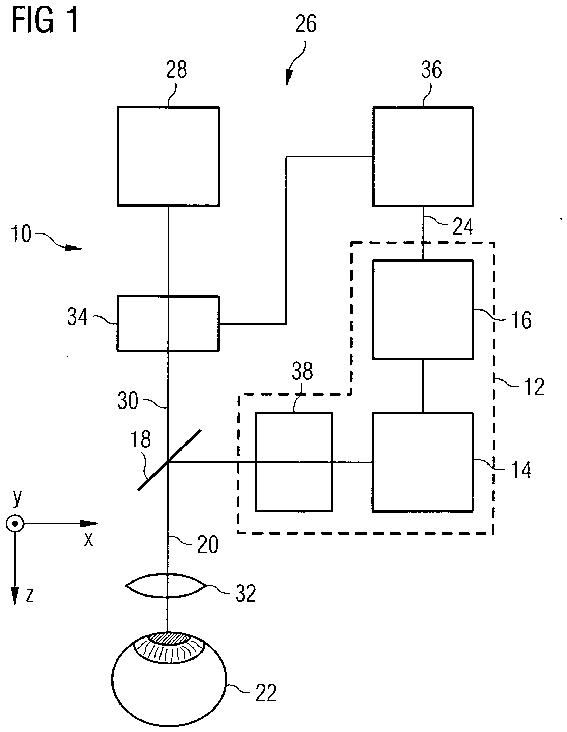

Figur 1 stellt stark schematisiert ein Ausführungsbeispiel eines Geräts zur Untersuchung oder Bearbeitung eines humanen Auges dar. Das Gerät ist im Allgemeinen mit 10 bezeichnet. Das Gerät umfasst einen Eye-Tracker 12. Der Eye-Tracker 12 umfasst eine interferometrische Bilderfassungseinrichtung 14 sowie ein mit der Bilderfassungseinrichtung 14 verbundenes Auswertemodul 16. Die interferometrische Bilderfassungseinrichtung 14 ist beispielsweise als eine OLCR-Messeinrichtung (OLCR: Optical Low Coherence Reflectometry) ausgebildet und sendet einen Messstrahl aus, der mittels eines (halbdurchlässigen oder dichroitischen) Umlenkspiegels 18 oder anderer geeigneter Strahlführungskomponenten längs eines optischen Strahlengangs 20 auf ein zu behandelndes Auge 22 gelangt. Der von der Bilderfassungseinrichtung 14 ausgesandte Messstrahl durchläuft einen Messscanner 38, welcher es ermöglicht, den Messstrahl abzulenken. Es ist somit eine externe sowie interne Abtastung des Auges 22 durch den Messstrahl an unterschiedlichen Stellen des Augengewebes möglich. Die Bilderfassungseinrichtung 14 bringt den erzeugten Messstrahl mit einem vom Auge 22 zurückkommenden Reflektionsstrahl in Interferenz. Aus den so gewonnenen Interferenzmessdaten können Schnittbilder des Auges 22 zeitaufgelöst erfasst werden. Die Bilderfassungseinrichtung 14 arbeitet dabei auf Basis einer zwei- oder drei-dimensionalen optischen Kohärenz-Tomographie. Das Auswertemodul 16 erhält von der interferometrischen Bilderfassungseinrichtung 14 Daten, welche die erfassten Schnittbilder umfassen und berechnet allein aus diesen Schnittbildern neben Position und Orientierung des Auges im drei-dimensionalen Raum auch die Bewegungen des Auges 22. Die Augenbewegungen stellen dabei Translationsbewegungen entlang der drei Raumrichtungen X, Y, Z sowie Rotationsbewegungen um die drei Raumachsen X, Y, Z dar. Ein eingezeichnetes Koordinatensystem veranschaulicht die drei Raumrichtungen X, Y, Z, wobei die Z-Achse die Richtung des Strahlengangs 20 definiert. Der Eye-Tracker 12 ergibt für die erfassten Augenbewegungen repräsentative Signale über die Schnittstelle 24 aus.

Das Gerät 10 umfasst ferner eine laserchirurgische Vorrichtung 26. Sie enthält einen Laser 28, welcher eine geeignet intensive (hochrepetierende oder Dauerstrich-) Laserstrahlung abgibt. Die Laserstrahlung breitet sich längs eines optischen Strahlengangs 30 aus und trifft dann auf das zu behandelnde Auge 22. In dem Strahlengang 30 sind verschiedene Komponenten zur Führung und Formung der Laserstrahlung angeordnet. Insbesondere umfassen diese Komponenten ein Fokussierobjektiv 32 sowie einen dem Objektiv 32 vorgeschalteten Scanner 34, mittels dessen der durch das Objektiv 32 erzeugte Fokus der von dem Laser 28 bereitgestellten Laserstrahlung entlang der X-, Y- und Z-Richtung ablenkbar ist. Eine Steueranordnung 36 steuert den Scanner 34 nach Maßgabe eines vorgegebenen Steuerprogramms, welches ein in dem Auge 22 zu erzeugendes Ablationsprofil implementiert. Im Bereich zwischen dem Spiegel 18 und dem Auge 22 verlaufen der Messstrahl der Bilderfassungseinrichtung 14 und der Behandlungs-Laserstrahl des Lasers 28 kollinear oder zumindest im Wesentlichen kollinear. Alternativ oder zusätzlich kann die laserchirurgische Vorrichtung 26 derart ausgebildet sein, dass der Laser 28 als ultrakurzpulsiger Laser ausgebildet ist, welcher gepulste Laserstrahlung mit Pulsdauern im Bereich von z.B. Piko-, Femto- oder Attosekunden abgibt und zum Schneiden innerhalb vom Augengewebe geeignet ist, wie es zum Beispiel für die LASIK oder bei einer Katarakt OP erforderlich ist. Die laserchirurgische Vorrichtung erlaubt beispielsweise Schnittgenauigkeiten von ±10 µm oder sogar ±1 µm.

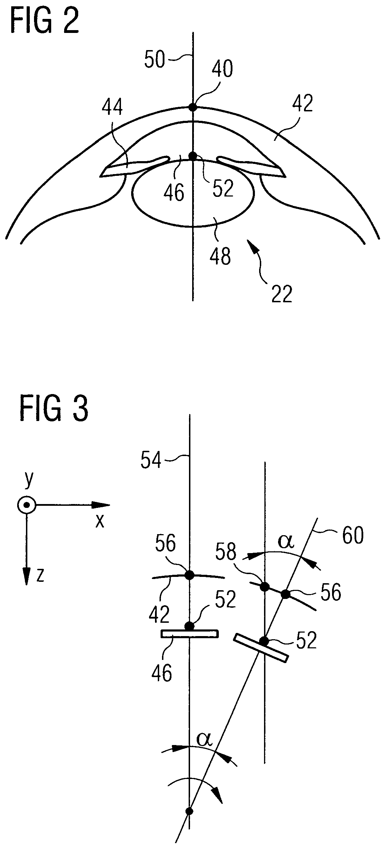

Ein von der Bilderfassungeinrichtung 14 erfasstes Schnittbild des Auges 22 ist beispielsweise in Figur 2 schematisch dargestellt. Auf diesem Bild sind der Apex (d.h. die vom Augenzentrum am weitesten entfernte Stelle) der Kornea 42 (Hornhaut), die Iris 44, die Pupille 46 und die Linse 48 zu erkennen. Zusätzlich ist in Figur 2 eine Achse 50 eingezeichnet, die beispielsweise die zentrale (optische) Achse des Auges 22 oder die Sehachse des Auges darstellt. Im vorliegenden Fall wird die Achse 50 durch die Position des Apex 40 und die Position der Pupillenmitte 52 dargestellt.

Zur Ablation von Augengewebe, muss der entsprechende Gewebeteil in Position und Orientierung zum Gerät 10 präzise ausgerichtet werden. Jedoch ist es nicht auszuschließen, dass sich das Auge während der Behandlung bezüglich des Koordinatensystems X, Y, Z (und somit bezüglich des Geräts 10) bewegt oder verdreht.

Im Stand der Technik sind Eye-Tracker bekannt, die auf der Positionserfassung der Pupillenmitte 52 der Pupille 46 im Auge 22 mit Hilfe von Kameras zur zwei-dimensionalen Erfassung der Position der Pupillenmitte 52 innerhalb der X-Y-Ebene beruhen. Ist es nun vorgesehen, Augengewebe an einer Position zu ablatieren, die von der Position der Pupillenmitte 52 verschieden ist, so kann es aufgrund von Augendrehungen zu suboptimalen Behandlungsergebnissen kommen, wie in Figur 3 näher erläutert.

Figur 3 zeigt eine schematische Darstellung der Pupille 46 sowie der Kornea 42 des Auges 22 in deren Position und Orientierung innerhalb des Koordinatensystems X, Y, Z. Die Gerade 54 verläuft hierbei parallel zur Z-Achse und entlang des optischen Strahlengangs 20. Soll nun an der Soll-Position 56 auf oder innerhalb (beispielsweise nach Freilegen korneainternen Gewebes durch beiseite Klappen einer Gewebelamelle (Flap) in der LASIK, i.e. Laser In Situ Keratomileusis) der Kornea 42 Augengewebe ablatiert werden, so führt eine Drehbewegung des Auges 22 in die durch die Gerade 60 dargestellte Orientierung des Auges 22 zu einer Abweichung der Ist-Position 58 der Ablation relativ zur Soll-Position 56 der Ablation, sofern -wie im Stand der Technik- das Eye-Tracking lediglich auf einer zwei-dimensionalen Erfassung der Pupillenmitte 52 beruht. Die Drehbewegung führt in diesem Fall zu einer transversalen Verschiebung der Soll-Position 56 gegenüber der Ist-Position 58 entlang der X- und Z-Achse.

Eine Orientierung des Ablationsvorgangs auf die Pupillenmitte kann daher unerwünscht sein. Bevorzugt kann dagegen eine Orientierung des Ablationsvorgangs an der Sehachse sein. Die Sehachse liegt nahe der optischen Achse des Auges und verläuft etwa durch den Apex der Kornea und die Linsenmitte der menschlichen Linse.

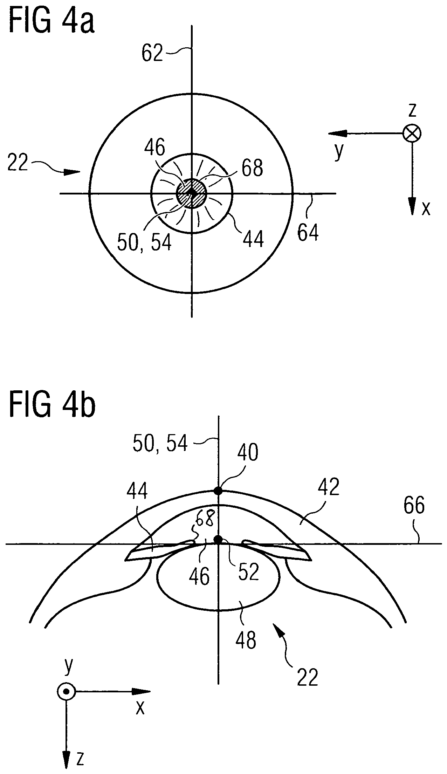

Das erfindungsgemäße Eye-Tracking beruht auf der zeitaufgelösten Erfassung von Schnittbildern des Auges durch interferometrische Bilderfassung auf Basis einer drei-dimensionalen optischen Kohärenz-Tomographie. Durch die drei-dimensionale Bildinformation kann das Auswertemodul 16 zeitaufgelöst die räumliche Lage sowie Orientierung des zu ablatierenden Augengewebeabschnitts oder zu bearbeitenden Augengewebeabschnitts (z.B. im Rahmen einer Katarakt-OP) sowie dessen Translations- und Rotationsbewegung ermitteln und ein für diese Daten repräsentatives Signal anhand der Schnittstelle 24 an die Steueranordnung 36 des Lasers 28 übermitteln, um den Fokusort der Behandlungs-Laserstrahlung abhängig von dem für die erfassten Daten repräsentativen Signal einzustellen. Desweiteren kann sich das erfindungsgemäße Eye-Tracking zur Steuerung des Fokusorts zum Beispiel am Apex 40 orientieren, dessen Position sich -im Unterschied zur Pupillenmitte 52- nicht bei wechselnder Beleuchtungssituation verändert. Außerdem bietet die Erfindung die Möglichkeit, aus den Schnittbildern ein Orientierungszentrum für die Laserablation zu wählen, das nah am zu behandelnden Gewebe positioniert ist, also zum Beispiel den Apex 40 als Orientierungszentrum für eine Behandlung der Kornea 42. Dabei ist es zweckmäßig, mindestens zwei zueinander orthogonale Schnittbilder 62, 64 des Auges 22 zu erfassen, die jeweils einen Schnitt im Wesentlichen entlang der Sehachse 50 oder einer sonstigen geeigneten Achse des Auges 22 repräsentieren, siehe Figur 4a. Alternativ oder zusätzlich ist es zweckmäßig mindestens einen Schnittbild 66 des Auges 22 zu erfassen, das einen Schnitt im Wesentlichen entlang des Randes 68 der Iris 44 (also des Pupillenrandes) des Auges 22 repräsentiert, siehe Figur 4b. Auch ist eine drei-dimensionale, insbesondere zeitaufgelöste Komplettbilderfassung (drei-dimensionale Tomographie) bestehend aus einer Vielzahl von Schnittbildern parallel zum Schnittbild 62, einer Vielzahl von Schnittbildern parallel zum Schnittbild 64 und/oder einer Vielzahl von Schnittbildern parallel zum Schnittbild 66 denkbar. Diese Komplettbilderfassung kann z.B. eine zeitaufgelöste 3D-Topographie der Kornea 42 repräsentieren, anhand der die translatorischen und rotatorischen Augenbewegungen ermittelt werden können und eine Orientierung der Behandlungs-Laserstrahlung an einem Punkt dieser 3D-Topographie (wie dem Apex 40 der Kornea 42) möglich ist.

## Patentansprüche

1. Gerät zur Untersuchung oder Bearbeitung eines humanen Auges, mit einem Eye-Tracker (12) zur Erfassung von Augenbewegungen und zur Ausgabe eines für die erfassten Augenbewegungen repräsentativen Signals, wobei der Eye-Tracker (12) eine zur zeitaufgelösten Erfassung von Schnittbildern des Auges eingerichtete interferometrische Bilderfassungseinrichtung (14), die auf Basis einer zwei- oder drei-dimensionalen optischen Kohärenz-Tomographie arbeitet, sowie ein die Augenbewegungen allein aus den Schnittbildern ermittelndes Auswertemodul umfasst,
**dadurch gekennzeichnet, dass** die interferometrische Bilderfassungseinrichtung (14) dazu eingerichtet ist, mindestens ein Schnittbild des Auges zu erfassen, das einen Schnitt entlang des Irisrandes des Auges repräsentiert.

2. Gerät nach Anspruch 1,
**dadurch gekennzeichnet, dass** die interferometrische Bilderfassungseinrichtung (14) dazu eingerichtet ist, mindestens zwei zueinander orthogonale Schnittbilder des Auges zu erfassen, die jeweils einen Schnitt im wesentlichen entlang der Sehachse des Auges repräsentieren.

3. Gerät nach Anspruch 1 oder 2,
**gekennzeichnet durch** Komponenten (28, 34) zur Bereitstellung von fokussierter Behandlungs-Laserstrahlung und zum Richten derselben auf das Auge sowie durch eine Steueranordnung (36), die dazu eingerichtet ist, den Fokusort der Behandlungs-Laserstrahlung abhängig von dem für die erfassten Augenbewegungen repräsentativen Signal einzustellen.

4. Verfahren zur Untersuchung eines humanen Auges, mit den Schritten:
Zeitaufgelöstes Erfassen von Schnittbildern des Auges auf Basis einer drei-dimensionalen optischen Kohärenz-Tomographie,
Ermitteln von Augenbewegungen allein aus den Schnittbildern, und
Ausgeben eines für die erfassten Augenbewegungen repräsentativen Signals,
**dadurch gekennzeichnet, dass** beim Erfassen der Schnittbilder mindestens ein Schnittbild des Auges erfasst wird, das einen Schnitt entlang des Irisrandes des Auges repräsentiert.

5. Verfahren nach Anspruch 4,
**dadurch gekennzeichnet, dass** beim Erfassen der Schnittbilder mindestens zwei zueinander orthogonale Schnittbilder des Auges erfasst werden, die jeweils einen Schnitt im wesentlichen entlang der Sehachse des Auges repräsentieren.

## Claims

1. Device for examining or treating a human eye, comprising an eye tracker (12) for capturing eye movements and for outputting a signal representative for the captured eye movements, wherein the eye tracker (12) comprises an interferometric image capturing apparatus (14) configured to capture, with a temporal resolution, slice images of the eye and operating on the basis of two-dimensional or three-dimensional optical coherence tomography, and an evaluation module ascertaining the eye movements from the slice images only,
**characterized in that** the interferometric image capturing apparatus (14) is configured to capture at least one slice image of the eye which represents a slice along the edge of the iris of the eye.

2. Device according to Claim 1,
**characterized in that** the interferometric image capturing apparatus (14) is configured to capture at least two mutually orthogonal slice images of the eye, said slice images each representing a slice substantially along the visual axis of the eye.

3. Device according to Claim 1 or 2,
**characterized by** components (28, 34) for the provision of focussed treatment laser radiation and for directing same onto the eye, and by a control arrangement (36) configured to set the focal location of the treatment laser radiation depending on the signal representative for the captured eye movements.

4. Method for examining a human eye, comprising the following steps:
capturing, with a temporal resolution, slice images of the eye on the basis of three-dimensional optical coherence tomography, ascertaining eye movements from the slice images only, and
outputting a signal representative for the captured eye movements,
**characterized in that** at least one slice image of the eye representing a slice along the edge of the iris of the eye is captured when capturing the slice images.

5. Method according to Claim 4,
**characterized in that** at least two mutually orthogonal slice images are captured when capturing the slice images, said mutually orthogonal slice images each representing a slice substantially along the visual axis of the eye.

## Revendications

1. Appareil pour l'examen ou le traitement d'un oeil humain, avec un eye-tracker (12) destiné à détecter des mouvements oculaires et à produire un signal représentatif des mouvements oculaires détectés, l'eye-tracker (12) comprenant un système d'acquisition d'images (14) interférométrique, aménagé pour la détection à résolution temporelle d'images en coupe de l'oeil, qui fonctionne sur la base d'une tomographie par cohérence optique bidimensionnelle ou tridimensionnelle, ainsi qu'un module d'analyse déterminant les mouvements oculaires uniquement à partir des images en coupe,
**caractérisé en ce que** le système d'acquisition d'images (14) interférométrique est aménagé pour détecter au moins une image en coupe de l'oeil qui représente une coupe le long du bord de l'iris de l'oeil.

2. Appareil selon la revendication 1,
**caractérisé en ce que** le système d'acquisition d'images (14) interférométrique est aménagé pour détecter au moins deux images en coupe de l'oeil orthogonales l'une par rapport à l'autre qui représentent respectivement une coupe essentiellement le long de l'axe optique de l'oeil.

3. Appareil selon la revendication 1 ou 2,
**caractérisé par** des composants (28, 34) destinés à fournir un rayonnement laser de traitement focalisé et à diriger ce rayonnement vers l'oeil, ainsi que par un dispositif de commande (36) qui est aménagé pour régler l'emplacement du foyer du rayonnement laser de traitement en fonction du signal représentatif des mouvements oculaires détectés.

4. Procédé pour l'examen d'un oeil humain, avec les étapes :
détection à résolution temporelle d'images en coupe de l'oeil sur la base d'une tomographie par cohérence optique tridimensionnelle,
détermination de mouvements oculaires uniquement à partir des images en coupe, et
production d'un signal représentatif des mouvements oculaires détectés,
**caractérisé en ce que**, lors de la détection des images en coupe, au moins une image en coupe de l'oeil est détectée, qui représente une coupe le long du bord de l'iris de l'oeil.

5. Procédé selon la revendication 4,
**caractérisé en ce que**, lors de la détection des images en coupe, au moins deux images en coupe de l'oeil orthogonales l'une par rapport à l'autre sont détectées, qui représentent respectivement une coupe essentiellement le long de l'axe optique de l'oeil.
